# EUROPÄISCHE PATENTSCHRIFT

(11) **EP 3 496 811 B1**
(45) Veröffentlichungstag und Bekanntmachung des Hinweises auf die Patenterteilung: **01.10.2025**
(21) Anmeldenummer: 17758814.2
(22) Anmeldetag: 09.08.2017
(51) Int. Cl.: A61N 5/06, A61L 2/00, A61L 2/025, A61L 2/14, A61L 2/10, A61M 1/06, A41C 3/04

(54) **TRAGBARE VORRICHTUNG ZUR DEKONTAMINATION EINER BRUST**
PORTABLE DEVICE FOR DECONTAMINATION OF A BREAST
DISPOSITIF PORTATIF DE DECONTAMINATION DE POITRINE FEMININE

(30) Priorität: 09.08.2016 EP 16183310
(43) Veröffentlichungstag der Anmeldung: 19.06.2019
(73) Patentinhaber: Medela AG, 6340 Baar (CH)
(72) Erfinder: HARTMANN, Peter, Gooseberry Hill Western Australia 6076 (AU); LARSSON, Michael, 6300 Zug (CH); LIMACHER, Kuno, 6312 Steinhausen (CH); BERNHARD, Jerome, 8003 Zürich (CH); CHRISTEN, Lukas, 6004 Luzern (CH); WIEDMER, Simona, 6300 Zug (CH); NAPOLETANO, Daniel, 8455 Rüdlingen (CH)
(74) Vertreter: Grünecker Patent- und Rechtsanwälte PartG mbB
(86) Internationale Anmeldenummer: PCT/EP2017/070250
(87) Internationale Veröffentlichungsnummer: WO 2018/029280

(56) Entgegenhaltungen:
- WO-A1-2006/037599
- WO-A1-2006/037599
- WO-A1-2015/088948
- WO-A1-2016/196395
- WO-A1-2016/196395
- WO-A2-2011/144344
- WO-A2-2011/144344
- CN-U- 203 915 153
- CN-U- 203 915 153
- CN-Y- 201 046 247
- CN-Y- 201 046 247
- JP-A- H0 596 018
- JP-A- H0 596 018
- US-A1- 2008 106 896
- US-A1- 2008 106 896
- US-A1- 2012 209 124
- US-A1- 2012 209 124

## Beschreibung

Die vorliegende Erfindung betrifft eine tragbare Vorrichtung zur Dekontamination einer Brust.

Eine Vorrichtung zur Dekontamination einer Brust ist beispielsweise aus der US 2003/0073930 A1 bekannt. Die in diesem Dokument beschriebene Vorrichtung weist eine Glocke auf, die über eine weibliche Brust eines Menschen gestülpt wird. Innerhalb der Glocke ist ein Druckelement vorgesehen, auf dessen Oberfläche ein absorbierendes Material angebracht ist. Durch den von dem Druckelement aufgebrachten Druck wird das absorbierende Material gegen die Brustwarze gedrückt, um entweder Feuchtigkeit von dieser aufzunehmen oder aber ein medizinisches Reagenz, wie beispielsweise ein fungizides oder antibakterielles Reagenz, aufzutragen. So wird beispielsweise eine Entzündung der Brust geheilt bzw. reduziert.

Bei der laktierenden Brust einer Mutter besteht eine erhöhte Gefahr einer Mastitis. Grund dafür ist, dass nach Entnahme von Milch, zum Beispiel durch Abpumpen oder durch den Säugling, die Milchgänge noch geöffnet sind, was zu einer Invasion von Krankheitserregern führen kann.

Zwar kann das aus der US 2003/0073930 A1 bekannte System die Gefahr einer Mastitis reduzieren, es kann jedoch vorkommen, dass die betreffende Patientin das Medikament, mit welchem das absorbierende Material getränkt ist, nicht verträgt.

Im Lichte dieser Problematik schlägt die vorliegende Erfindung eine tragbare Vorrichtung zur Dekontamination einer Brust mit den Merkmalen von Anspruch 1 vor.

Die tragbare Dekontaminationsvorrichtung zeichnet sich insbesondere dadurch aus, dass die Dekontaminationseinheit die Brust mittels einer physikalischen Methode dekontaminiert.

Dadurch, dass eine solche physikalische Methode verwendet wird, braucht keine flüssige oder pastöse Substanz zur Dekontamination verwendet werden. Im Gegensatz zu biologischen oder chemischen Dekontaminationsverfahren wird vorliegend zumindest eine physikalische Dekontamination durchgeführt. Eine physikalische Größe wird demnach als Mittel der Kontamination verwendet. Diese Größe wird zum Beispiel mittels elektrischer Energie erzeugt und die Brust wird der mittels elektrischer Energie erzeugten Größe ausgesetzt.

Vorteilhaft bei einer solchen physikalischen Dekontamination ist, dass diese nicht lediglich gegen einzelne Spezies von Mikroorganismen wirkt, sondern "breitbandig" gegen eine Vielzahl von Mikroorganismen. Der Begriff "Mikroorganismus" wird in diesem Zusammenhang wie folgt verwendet. Mikroorganismen können mikroskopisch kleine Lebewesen (Organismen) sein, die als Einzelwesen nicht mit bloßem Auge erkennbar sind. Sie werden auch als Mikroben oder Kleinstlebewesen bezeichnet. Sie bilden im System der Lebewesen keine einheitliche Gruppe. Zu den Mikroorganismen zählen Bakterien (z. B. Milchsäurebakterien), Pilze (z. B. Backhefe), mikroskopische Algen (z. B. Chlorellen) sowie Protozoen (z. B. Pantoffeltierchen und der Malaria-Erreger Plasmodium). Vorliegend werden auch Viren zu den Mikroorganismen gerechnet. Viren werden zwar überwiegend nicht als Lebewesen, also auch nicht als Mikroorganismen angesehen. Gelegentlich werden sie aber dennoch zu den Mikroorganismen gezählt, und dann wird dementsprechend die Virenforschung (Virologie) als ein Teilgebiet der Mikrobiologie angesehen.

Zudem bringt eine solche physikalische Dekontamination den Vorteil, dass keine Resistenzen auftreten, wie es der Fall ist, wenn z.B. Antibiotika (chemische Reagenzien) zur Dekontamination verwendet werden.

Auch ist die Behandlung während der Zeit des Säugens von Kindern möglich, da die Brustwarze rückstandsfrei für die Kinder zur Nahrungsaufnahme zur Verfügung steht.

Weiterer relevanter Stand der Technik ist in folgenden Dokumenten offenbart: WO 2006/037599 A1, US 2008/106896 A1, WO 2011/144344 A2, WO 2016/196395 A1, CN 201 046 247 Y, CN 203 915 153 U, JP H05 96018 A, US 2012/209124 A1 und WO 2015/088948 A1.

Zur Lösung des vorbeschriebenen Problems wird eine tragbare Vorrichtung zur Dekontamination einer Brust mit den Merkmalen von Anspruch 1 angegeben. Weitere vorteilhafte Ausgestaltungen sind in den anhängigen Ansprüchen definiert.

Nachfolgend werden einige Varianten beschrieben, die hilfreich zum Verständnis der Erfindung sind. Die erfindungsgemäße tragbare Vorrichtung zur Dekontamination einer Brust zeichnet sich dadurch aus, dass die Dekontaminationseinheit in einem Pad bzw. Kissen aus einem sich verformbaren Material vorgesehen ist, welches ein separates Element in den Büstenhalter einlegbar ist und zumindest eine Dekontaminationseinheit auf einer der Brust zugewandten Seite und zumindest eine Dekontaminationseinheit auf einer der Brust abgewandten Seite vorgesehen ist.

Gemäß einer vorteilhaften Weiterbildung der Erfindung kann diese Dekontaminationseinheit zumindest eines der folgenden Elemente enthalten: eine Lichtquelle, insbesondere UV-Lichtquelle, eine Ultraschallquelle, eine Ozonisierungsquelle, eine Plasmaquelle, insbesondere Kaltplasmaquelle. Diese Quellen erzeugen alle eine energetische/hochenergetische Strahlung und/oder energetische/hochenergetische Teilchen und/oder energetische/hochenergetische Moleküle und/oder energetische/hochenergetische Wellen, welche zur Dekontamination mit entsprechenden Erregern, wie Bakterien und Viren, wechselwirken, um diese unschädlich zu machen. Zur Erzeugung dieser Strahlung bzw. Teilchen wird z.B. eine Energiequelle, insbesondere elektrische Energiequelle, verwendet.

Als Lichtquelle kann eine LED, insbesondere eine UV-LED, Verwendung finden. Als Ultraschallquelle kann beispielsweise ein piezokeramisches Element Verwendung finden. Beide zuvor genannten Elemente können heutzutage sehr klein gebaut werden und können in unmittelbarer Nähe der zu dekontaminierenden Stelle der Brust montiert werden.

Insbesondere wird als Lichtquelle bzw. Erregungsstrahlungsquelle eine UV-C-Lichtquelle verwendet. Solches UVC-Licht wird auch als fernes UV-Licht bezeichnet und grenzt sich vom nahen UV-Licht (UV-A bzw. Schwarzlicht) und mittleren UV-Licht (UV-B) über die Wellenlänge ab.

Insbesondere weist das UVC-Licht eine Wellenlänge von 280 bis 100 nm auf und eine Photonenenergie von 4,43 eV bis 12,4 eV. Das UV-C Licht kann noch einmal in UVC-FUV mit einem Wellenlängenbereich von 280 bis 200 nm und einer Photonenenergie von 4,43 eV bis 6,20 eV und UVC-VUV mit einem Wellenlängenbereich von 200 nm bis 100 nm und einer Photonenenergie von 6,20 eV bis 12,4 eV unterteilt werden. Unterhalb von 200 nm (UVC-VUV) ist die Ultraviolettstrahlung so kurzwellig und energiereich, dass sie durch molekularen Sauerstoff absorbiert wird. Dabei wird der molekulare Sauerstoff in zwei freie Sauerstoffradikale gespalten, die jeweils mit einem weiteren Molekül Sauerstoff zu Ozon weiter reagieren.

Soweit vorliegend UVC-VUV-Strahlung verwendet wird, wird neben der Dekontamination durch die Strahlung selbst auch eine Dekontamination durch das dabei entstehende Ozon erzeugt.

Das UV-C-Licht dringt aufgrund der relativ kurzen Wellenlänge und damit hohen Streuung nicht sehr tief in die Haut ein. Während beispielsweise bei 280 nm die Aminosäure Tryptophan beschädigt bzw. degeneriert wird, werden bei 265 nm die Nukleinsäuren am stärksten beschädigt.

Vorzugsweise werden demnach bei der vorliegenden Erfindung folgende Wellenlängenbereiche an Strahlung verwendet.

Gemäß einer Variante die hilfreich zum Verständnis der Erfindung ist kann die Vorrichtung eine glockenartige Struktur aufweisen, welche über die Brust stülpbar ist und diese zumindest bereichsweise umgibt. Diese glockenartige Struktur kann demnach über die Brust gestülpt werden. Die glockenartige Struktur kann in diesem Fall schon so vorgeformt sein, dass diese an die Brust anlegbar ist. Innerhalb dieser glockenartigen Struktur kann dann die entsprechende Dekontaminationseinheit vorgesehen sein. Durch die glockenartige Struktur wird gewährleistet, dass der Bereich der Brust der dekontaminiert werden soll, von der äußeren Umgebungsatmosphäre abgeschlossen ist, und der Bereich der Brustoberfläche innerhalb der Glocke effizient dekontaminiert wird. Diese glockenartige Struktur kann eine Art Kappe sein, welche über die Brust gestülpt wird.

Die glockenartige Struktur kann im Wesentlichen durch einen vorderen, zur Brust hin gerichteten aufgeweiteten Abschnitt gebildet werden, der nach Art eines Trichters ausgebildet ist. Von der von der Brust abgewandten Seite des aufgeweiteten Abschnitts ragt z.B. nach hinten ein zylinderförmiger Tubus ab. Dieser Tubus wird auch als konische Verjüngung bezeichnet und weist bevorzugt einen Durchmesser auf, der geringer ist als die Öffnung des aufgeweiteten Abschnitts, die über die Brust gestülpt wird. Die glockenartige Struktur und die sich bevorzugt daran anschließende konische Verjüngung können integral und aus einem einheitlichen Material ausgebildet sin. Diese Elemente können auch mehrteilig ausgebildet sein.

Die glockenartige Struktur kann so ausgebildet sein, das sich diese erst beim Anlegen an die Brust instantan ausbildet und vorzugsweise bei Entfernung von der Brust wieder in deren Grundzustand zurückkehrt. Die glockenartige Struktur kann auch so ausgebildet sein, das sie in einem nicht an der Brust angelegten Grundzustand z.B. als flaches Gebilde, das keine glockenartige Struktur aufweist, ausgebildet ist. Erst bei Anlegen an die Brust bildet sich die glockenartige Struktur instantan aus. Bei Entfernung von der Brust kehrt das Element wieder in seinen Grundzustand zurück.

Die glockenartige Struktur kann so ausgebildet sein, dass die Brust in dieser konturgenau zur Anlage kommt und im Bereich der Brustwarze an deren Innenseite eine Ausnehmung ausgebildet ist. Durch die konturgenaue Anlage ist es möglich, die Dekontaminationseinheit als einzelnes Element an einer wohldefinierten Stelle und in direkter Nähe zu dem zu behandelnden Bereich der Brust zu platzieren, ohne dass Bereiche auf der Brustoberfläche, die nicht dekontaminiert werden sollen, mittels der energetischen Strahlung bzw. Teilchen beaufschlagt werden.

Gemäß einer Variante die hilfreich zum Verständnis der Erfindung ist, kann die Dekontaminationseinheit kann z.B. ein Gehäuse aufweisen, in welchem eine oder mehrere Erregungsstrahlungsquellen vorgesehen sind. Das Gehäuse kann ein separates Element sein, welches lösbar an der Vorrichtung montierbar ist. Dies hat den Vorteil, dass wenn die glockenartige Struktur oder weitere Teile der Vorrichtung mit Wasser abgespült werden, die Dekontaminationseinheit, in der beispielsweise auch Elektronik vorgesehen ist, nicht mit Wasser in Kontakt kommt. Zudem hat diese Ausgestaltung den Vorteil, dass die Dekontaminationseinheit auch an verschieden ausgestalteten glockenartigen Strukturen angebracht werden kann.

Die Dekontaminationseinheit kann z. B. eine oder auch mehrere Erregungsstrahlungsquellen enthalten. Diese Erregungsstrahlungsquelle ist eine Quelle, mit welcher eine physikalische Dekontamination bewirkt wird. Eine solche Erregungsstrahlungsquelle kann beispielsweise eine UV-Strahlungsquelle, insbesondere eine UV-C-Strahlungsquelle, eine Plasma-Strahlungsquelle, eine Ultraschall-Strahlungsquelle oder eine Ozonisierungsquelle sein.

Die Dekontaminationseinheit kann mit deren Gehäuse so ausgestaltet sein, dass das Gehäuse durch Einrasten an der Vorrichtung zur Dekontamination der Brust befestigt werden kann, insbesondere an einem Abschnitt eines aufgeweiteten Bereiches, welcher über die Brust stülpbar ist, und/oder an einem Abschnitt eines zylinderförmigen Tubus, welcher sich aus der brustabgewandten Seite von dem aufgeweiteten Bereich absteht.

So ist eine besonders einfache Montage und Demontage der Dekontaminationseinheit möglich.

Bei der Vorrichtung kann lediglich an dem Gehäuse eine Elektronik zum Betrieb der Dekontaminationseinheit angebunden sein. Die Vorrichtung zur Dekontamination der Brust mag außer an dem Gehäuse keine weitere Quelle für Erregerstrahlung aufweisen.

Es hat sich als günstig herausgestellt, dass weder an dem aufgeweiteten Abschnitt noch an dem zylinderförmigen Tubus selber eine Erregungsstrahlungsquelle vorgesehen ist. Es ist vorteilhaft, die Erregungsstrahlungsquelle bzw. die Erregungsstrahlungsquellen lediglich in dem als separates Element ausgebildeten Gehäuse der Dekontaminationseinheit vorzusehen. So wird gewährleistet, dass z. B. an der Vorrichtung (mit Ausnahme des separaten Gehäuses der Dekontaminationseinheit) keine Elektronik, zumindest keine Elektronik zum Betrieb der Dekontaminationseinheit vorgesehen ist.

Das Gehäuse der Dekontaminationseinheit kann den aufgeweiteten Bereich und/oder den zylinderförmigen Tubus radial außen umgeben. So wird eine einfache Montage gewährleistet. Insbesondere kann es günstig sein, das Gehäuse der Dekontaminationseinheit als eine Art Manschette auszubilden, die um den zylinderförmigen Tubus und/oder den aufgeweiteten Bereich gelegt wird.

Insbesondere kann diese Manschette einen C-förmigen Querschnitt aufweisen, um auf den zylinderförmigen Tubus mittels Schnappverbindung und damit bevorzugt formschlüssig befestigt werden.

Es hat sich als günstig herausgestellt, dass der aufgeweitete Bereich und/oder der zylinderförmige Tubus zumindest in einem Abschnitt, in dem, bei an diesem montierter Dekontaminationseinheit, eine Erregerstrahlungsquelle platziert ist, für Erregerstrahlung der Dekontaminationseinheit transparent, insbesondere UV-C transparent ausgebildet ist.

Es ist auch denkbar, dass das Gehäuse der Dekontaminationseinheit in den aufgeweiteten Bereich und/oder den zylinderförmigen Tubus eingeschoben ist und über ein außen an dem aufgeweiteten Bereich und/oder den zylinderförmigen Tubus platziertes Induktionselement betreibbar ist.

Das Induktionselement kann integral an der glockenartigen Struktur, oder aber auch als separates Element vorgesehen sein. Zur Montage und zum Betrieb der Dekontaminationseinheit wird also in einem ersten Schritt die Manschette mit der Erregungsstrahlungsquelle in den aufgeweiteten Bereich und/oder den zylinderförmigen Tubus eingeschoben und dann von außen an dem aufgeweiteten Bereich und/oder dem zylinderförmigen Tubus das Induktionselement montiert. Dieses Induktionselement kann auch nach Art einer Manschette ausgestaltet sein. Es kann auch form- und/oder kraftschlüssig, beispielsweise über Magnete befestigt werden.

Die Dekontaminationseinheit kann auch eine Erregerstrahlungsquelle aufweisen, welche integral und unverlierbar an einem Basisteil vorgesehen ist. Dieses Basisteil kann an die glockenartige Struktur ankoppelbar sein. Es können Leitungen zum Betrieb der Erregerstrahlungsquelle durch das Basisteil geführt sein, und außen können an dem Basisteil Kontaktelemente vorgesehen sein, über welche eine Spannungsversorgung anschließbar ist.

Im Bereich der Kontaktelemente können Mittel vorgesehen sind, über welche ein Batteriepack wiederholt an- und abmontiert und mit den Kontaktelementen verbunden werden kann.

Die Dekontaminationseinheit kann eine Erregerstrahlungsquelle aufweisen, welche integral an dem Basisteil angeordnet ist, wobei das Basisteil lösbar rückseitig an der glockenartigen Struktur montierbar ist.

Die Dekontaminationseinheit kann gemäß einer Variante die hilfreich zum Verständnis der Erfindung ist nach Montage der Vorrichtung an der Brust anliegen. Die Dekontaminationseinheit kann so vorzugsweise eine Fläche aufweisen, die unmittelbar an der zu behandelnden Oberfläche anliegt. Soweit die glockenartige Struktur im Bereich der Brustwarze an der Innenseite eine Ausnehmung bildet, kann die Innenoberfläche, insbesondere die gesamte Innenoberfläche, dieser Ausnehmung zum Beispiel durch die Dekontaminationseinheit gebildet werden. Alternativ können auch in dieser Ausnehmung eine Mehrzahl einzelner Dekontaminationseinheiten vorgesehen sein, jeweils für sich an der Brustwarze anliegend ausgebildet sind.

Gemäß einer vorteilhaften Weiterbildung der Erfindung können zumindest zwei Dekontaminationseinheiten an verschiedenen Positionen vorgesehen sein. So können eine oder mehrere Dekontaminationseinheit/en in der Ausnehmung und/oder auch eine oder mehrere Dekontaminationseinheit/en in der glockenartigen Struktur angebracht sein. Diese sind zum Beispiel so angeordnet, dass der Warzenhof dekontaminiert werden kann. Eine oder mehrere Dekontaminationseinheiten können so angeordnet sein, dass diese oder eine Gruppe dieser die unterschiedlichen Regionen der Brust, nämlich die Brustwarze, den Warzenhof oder eine sonstige Brustoberfläche selektiv dekontaminieren. Diese Dekontaminationseinheiten können untereinander unterschiedliche Intensitäten beziehungsweise Stärken von Dekontaminationsintensität aufweisen, welche auf die unterschiedlichen Bereiche abgestimmt sind. So können unterschiedliche Bereiche der Brust selektiv dekontaminiert werden.

Gemäß einer vorteilhaften Weiterbildung der Erfindung können die Dekontaminationseinheit beziehungsweise die Dekontaminationseinheiten derart positioniert sein, dass diese bei Montage der Vorrichtung an der Brust die Brustwarze und/oder den Warzenhof physikalisch dekontaminieren. Zudem kann diese bei Montage der Vorrichtung an der Brust zusätzlich auch den Warzenhof dekontaminieren. Wie bereits beschrieben, können in diesem Fall die unterschiedlichen Dekontaminationseinheiten, soweit sie beispielsweise die Brustwarze oder den Warzenhof dekontaminieren, eine unterschiedliche Dekontaminationsintensität haben. Eine solche unterschiedliche Dekontaminationsintensität kann über die Wellenlänge der Lichtstrahlung, soweit es sich um eine Lichtquelle handelt, die Energie einer Ultraschallquelle oder die Menge an generiertem Ozon beziehungsweise Plasma eingestellt werden.

Erfindungsgemäß handelt es sich bei der tragbaren Vorrichtung um ein Einlagepad, insbesondere Einlegekissen. Alternativ kann es sich gemäß einer Variante die hilfreich zum Verständnis der Erfindung ist, um eine elektrisch betriebene oder handbetriebene Milchpumpe handeln. Eine tragbare Vorrichtung ist eine Vorrichtung, die so leicht getragen werden kann, dass sie von einer einzigen Person ohne großen Aufwand und unter verschiedenen Umständen und an verschiedenen Orten verwendet werden kann. Sie kann besonders kompakt ausgebildet sein. Die gesamte Vorrichtung überschreitet vorzugsweise nicht das Gewicht von zwei Kilogramm, vorzugsweise einem Kilogramm. Soweit es sich um einen Büstenhalter handelt, kommen alle möglichen bekannten Arten von Büstenhaltern in Betracht. Milchpumpen werden zum Beispiel verwendet, um Milch von der weiblichen Brust abzupumpen.

Gemäß einer vorteilhaften Weiterbildung der Erfindung kann die Dekontaminationseinheit an eine autonome Energieversorgung angeschlossen sein, welche in der Vorrichtung integriert ist. Soweit es sich bei der Vorrichtung demnach um einen Büstenhalter, eine handbetriebene oder elektrisch betriebene Milchpumpe handelt, kann die Energieversorgung in der jeweiligen Vorrichtung integriert sein. Eine solche Energieversorgung kann eine wiederaufladbare Batterie oder ein Akkumulator sein. Eine solche Energieversorgung kann jedoch auch ein Transformator sein, der die Netzspannung in eine niedrigere Energieversorgungsspannung umwandelt. Vorzugsweise ist die Energieversorgung mit der Energieerzeugung kombiniert, d.h., es handelt sich um eine wiederaufladbare Batterie oder um einen wiederaufladbaren Akkumulator.

Erfindungsgemäß ist die Dekontaminationseinheit in einem Pad oder Kissen vorgesehen, welches als separates Element in einen Büstenhalter einlegbar ist, oder die Dekontaminationseinheit kann fest an der Vorrichtung montiert sein. Ein Kissen wird als schwammartig verformbares softes Pad verstanden. Soweit es sich um ein Pad handelt, in dem die Dekontaminationseinheit integriert ist, kann dieses beispielsweise in in dem Büstenhalter vorgesehene Taschen eingesetzt werden. Das Pad bzw. Kissen kann aber auch zwischen Brust und Büstenhalter geklemmt werden ohne dass es z.B. in eine solche Tasche eingesetzt wird. Das Pad selbst weist dementsprechend die physikalische Dekontaminationseinheit auf. Das Pad kann eine separate autonome Einheit bilden, soweit die Energieversorgung und Energieerzeugung darin integriert ist. Im Falle der Verwendung der Vorrichtung in einer Milchpumpe kann die Dekontaminationseinheit vorzugsweise fest mit dieser verbunden sein. Dann ist gewährleistet, dass die Dekontaminationseinheit in vorbestimmter Anordnung zur Brust ausgerichtet ist, zum Beispiel wenn die Dekontaminationseinheit rückseitig innerhalb der glockenartigen Struktur, die einen Saugstutzen für die Brust bildet, angebracht ist. Dieses Pad bzw. Kissen kann auch einen Schlauchstutzen aufweisen über welchen eine Pumpe anschließbar ist, um Milch von der Brust abzupumpen.

Anstelle der pad- bzw. kissenartigen Struktur kann gemäß einer nicht beanspruchten Variante die glockenartige Struktur auch aus einem formstabilen z.B. harten Material ausgebildet sein, welches insbesondere derart konfiguriert ist, dass es bei Anlegen eines Vakuums nicht bzw. nur unwesentlich kollabiert. Damit ein Vakuum angelegt werden kann, ist es günstig, dass die glockenartige Struktur vakuumdicht ist, und auch dichtend an die Brust angelegt werden kann. Die glockenartige Struktur kann so durch eine Art formstabile Brusthaube gebildet werden.

Erfindungsgemäß ist jeweils eine Dekontaminationseinheit auf einer der Brust zugewandten Seite und auf einer der Brust abgewandten Seite der Vorrichtung vorgesehen.

Gemäß einer vorteilhaften Weiterbildung der Erfindung kann die Vorrichtung eine Steuereinrichtung aufweisen, welche die Dekontaminationseinheit ein- und ausschaltet und/oder die Intensität reguliert. So kann in der Vorrichtung eine Steuereinrichtung integriert sein, mittels welcher die Dekontaminationseinheit beziehungsweise die Dekontaminationseinheiten gesteuert werden. Eine solche Steuereinrichtung kann ein simpler An-/Ausschalter sein, der zwischen der Spannungsversorgung der Dekontaminationseinheit vorgesehen ist, oder aber ein Mikrocomputer, der in Abhängigkeit der Notwendigkeit nach Anbringen der Vorrichtung an der Brust die Dekontaminationseinheit beziehungsweise die Dekontaminationseinheiten steuert. So kann beispielsweise mittels eines Timers bestimmt werden, wann die Pumpe angesetzt wurde, und daraus bestimmt werden, wann die Dekontaminationseinheit eingeschaltet werden soll. Diese Steuereinrichtung kann z.B., soweit mehrere Dekontaminationseinheiten vorgesehen sind, diese selektiv steuern und/oder die Intensität der Dekontaminationsleistung in Abhängigkeit der Region, in welcher die Dekontaminationseinheit an der Vorrichtung vorgesehen sind, variieren.

Gemäß einem nebengeordneten Aspekt nach Anspruch 12 betrifft die Erfindung auch den Betrieb einer tragbaren Vorrichtung zur Dekontamination einer Brust, wie sie zuvor beschrieben ist. Das Verfahren zeichnet sich dadurch aus, dass die Dekontaminationseinheit zur Abtötung von Mikroorganismen eingeschaltet wird, um zu verhindern, dass diese in die Milchgänge der Brust eintreten. Vorteilhafterweise wird der Betrieb der Vorrichtung nach Montage dieser Vorrichtung an der Brust eines weiblichen Menschen durchgeführt.

Weitere vorteilhafte Ausgestaltungen der Erfindung ergeben sich aus den nachfolgend erläuterten Beispielen in Verbindung mit der Zeichnung. In dieser zeigen:
- Figur 1: ein erstes Beispiel einer handbetriebenen Milchpumpe mit einer Dekontaminationseinheit,
- Figur 2 a, 2b, 2c: ein zweites Beispiel einer elektrisch betriebenen Milchpumpe, wobei Figur 2b den eingekreisten Bereich in Figur 2c vergrößert zeigt;
- Figur 3a: ein drittes Beispiel, wobei der Aufsatz für eine Brust als glockenförmiger Aufsatz ausgebildet ist,
- Figuren 3b und 3c: eine Querschnittsansicht des Beispiels aus Figur 3a,
- Figuren 4a und 4b: ein in einen Büstenhalter eingelegtes Pad bzw. Kissen in dessen Querschnittsansicht, wobei Figur 4b den eingekreisten Bereich in Figur 4a vergrößert zeigt;
- Figur 5: eine Spezialvorrichtung, die lediglich zur Dekontamination der Brust verwendet wird,
- Figur 6: ein weiteres Beispiel einer alternativen Spezialvorrichtung, die lediglich zur Dekontamination der Brust verwendet wird,
- Figur 7a: ein viertes Beispiel, bei dem die Dekontaminationseinheit als Manschette außen um die glockenartige Struktur und die sich daran anschließende konische Verjüngung gelegt ist,
- Figur 7b: eine Querschnittsansicht des Beispiels aus Figur 7a entlang der Linie A-A,
- Figur 7c und 7d: eine Aufsicht auf das Beispiel aus Figur 7a von schräg oben, wobei Figur 7d den eingekreisten Bereich in Figur 7c vergrößert zeigt;
- Figur 8a: ein fünftes Beispiel, bei dem die Dekontaminationseinheit im Inneren der konischen Verjüngung eingesetzt ist,
- Figur 8b: eine Querschnittsansicht des Beispiels aus Figur 8a entlang der Linie A-A,
- Figur 8c und 8d: eine Aufsicht auf das Beispiel aus Figur 8a von schräg oben, wobei Figur 8d den eingekreisten Bereich in Figur 8c vergrößert zeigt;
- Figur 9a: ein sechstes Beispiel, bei dem ein Batteriepack wiederholt lösbar rückseitig an der Dekontaminationsvorrichtung vorgesehen ist,
- Figur 9b: eine Querschnittsansicht des Beispiels aus Figur 9a entlang der Linie G-G,
- Figur 10a: ein sechstes Beispiel, bei dem die glockenartige Struktur vorderseitig auf die Dekontaminationseinheit aufgesetzt ist,
- Figur 10b: eine Querschnittsansicht des Beispiels aus Figur 9a entlang der Linie A-A, und
- Figuren 11a und b: ein siebtes Beispiel, bei dem die Dekontaminationseinheit als Kissen bzw. Pad ausgebildet ist, wobei in dem Pad des Weiteren ein Schlauchstutzen zum Anschluss an eine Pumpe vorgesehen ist und wobei Figur 11b den eingekreisten Bereich in Figur 11a vergrößert zeigt.

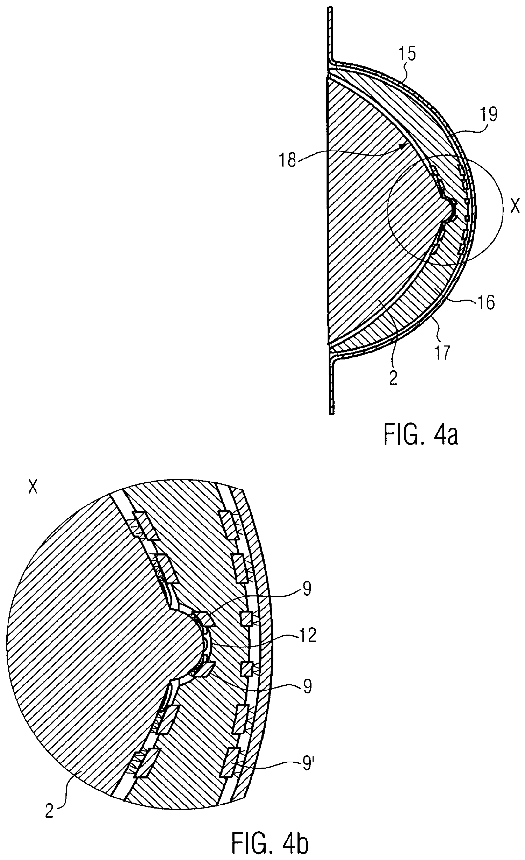

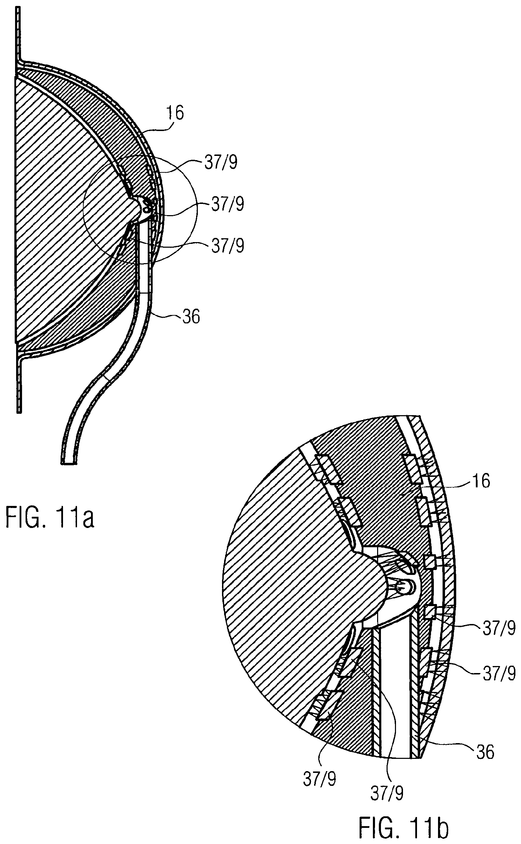

Gemäß einer von den zuvor beschriebenen Beispielen in den Figuren sind die Beispiele gemäß Figuren 4 und 11 Ausführungsbeispiele der vorliegenden Erfindung. Die weiteren Beispiele sind Varianten, die hilfreich zur vorliegenden Erfindung sind.

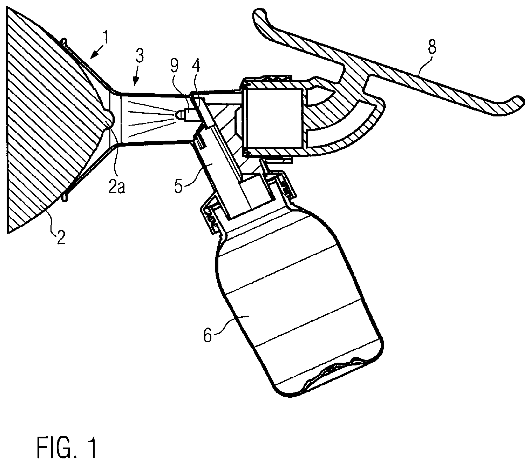

Die in Figur 1 dargestellte handbetriebene Milchpumpe ist ein Beispiel einer tragbaren Vorrichtung zur Dekontamination einer Brust 2. Diese handbetriebene Milchpumpe weist eine glockenartige Struktur 1 auf, die einen Bereich der Brust 2 dichtend umschließt, so dass aus dem Milchkanal der Brust 2 Milch herausgesogen werden kann. Die Brustwarze 2a ist in einem Bereich der glockenartigen Struktur 1 positioniert, wo diese eine konische, rohrartige Verjüngung 3 aufweist. Die Brustwarze 2a ist vorteilhafterweise somit beim Abpumpen in einem Bereich der rohrartigen Verjüngung 3 vorgesehen. Die konische, rohrartige Verjüngung 3 weist an deren der Brustwarze 2a gegenüber liegenden Seite 4 eine Leitung 5 auf, die zu einem Auffangbehälter 6 führt. Die glockenartige Struktur 1 ist integral mit der Leitung 5 und einem Schraubdeckel 7 verbunden und bildet somit eine Aufsatzeinheit, welche auf den Auffangbehälter 6 aufgeschraubt werden kann. Zudem ist an dieser Aufsatzeinheit ein Hebel 8 vorgesehen, über welchen ein Vakuum in der glockenartigen Struktur 1 und in dem Auffangbehälter 6 generiert werden kann.

An einer Wand an der der Brustwarze 2a gegenüber liegenden Seite 4 der konischen, rohrartigen Verjüngung 3 ist eine Dekontaminationseinheit 9 vorgesehen. Diese ist im vorliegenden Fall eine UV-Leuchtdiode (UV-LED), deren UV Licht auf die Brustwarze 2a und zumindest teilweise auf den um die Brustwarze herum ausgebildeten Warzenhof 10 abstrahlt.

Die UV-Leuchtdiode wird über einen in Figur 1 nicht dargestellten, in die Aufsatzeinheit einsetzbaren Akku, welcher ein Standardmaß aufweist, mit Energie versorgt. In der Aufsatzeinheit befindet sich zudem eine nicht gezeigte Steuereinrichtung, über welche die UV-Leuchtdiode an- und ausgestellt und/oder reguliert werden kann. Alternativ zu der Stromversorgung mittels eines Ackumulators ist eine Stromversorgung mittels einer Batterie (nicht wiederaufladbar) denkbar. Alternativ kann auch ein Kondensator durch Betätigung des Hebels 8 aufgeladen werden, der die Stromversorgung für die UV-Leuchtdiode bereitstellt. Es kann des Weiteren in der Aufsatzeinheit eine Steuervorrichtung vorgesehen sein, die den Betrieb der Leuchtdiode derart steuert, dass diese beim Milchabpumpen oder lediglich kurz nach dem das Milchabpumpen beendet ist, angestellt wird. Auch kann die glockenartige Struktur 1 einen Kontaktsensor aufweisen, so dass die Leuchtdiode lediglich dann arbeitet, wenn die glockenartige Struktur 1 an der Brust anliegt. Diese Ausgestaltung bietet eine erhöhte Sicherheit, insbesondere wenn es sich um eine Dekontaminationsvorrichtung auf UV-Basis handelt, da so verhindert wird, dass ein Betrieb stattfindet, wenn eine Person in die Dekontaminationseinheit hereinschauen kann.

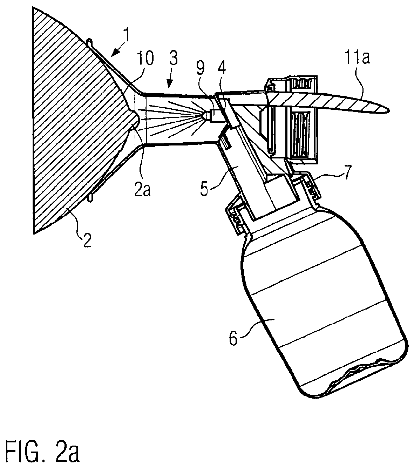

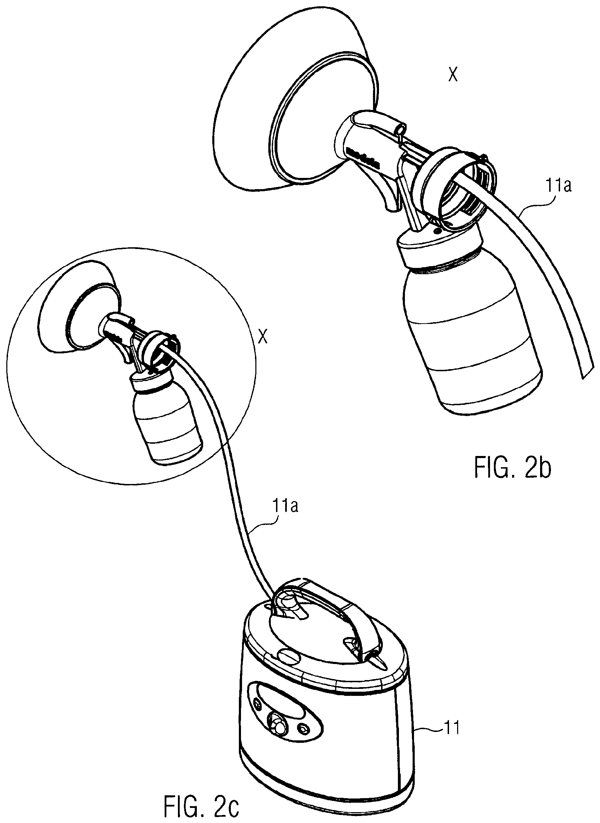

Ein alternatives Beispiel einer Milchpumpe ist in Figuren 2a, 2b und 2c gezeigt. Die Pumpe selbst ist gleich aufgebaut wie die Pumpe in Figur 1. Deshalb wird auf eine erneute Beschreibung der einzelnen Elemente verzichtet. Gleiche Elemente sind mit den gleichen Bezugszeichen versehen.

Im Unterschied zu der Pumpe aus Figur 1, handelt es sich bei dem Ausführungsbeispiel in Figuren 2a, 2b und 2c nicht um eine handbetriebene Pumpe, sondern um eine elektrisch betriebene Pumpe.

Wie in Figuren 2a und 2b zu erkennen ist, ist eine Vakuumerzeugungsstation 11 vorgesehen, die separat von der Aufsatzeinheit und dem Auffangbehälter 6 vorgesehen ist. Die Aufsatzeinheit mit dem Auffangbehälter 6 ist über den Vakuumschlauch 11a an die Vakuumerzeugungsstation 11 angebunden. Eine Querschnittsansicht der Aufsatzeinheit und des Auffangbehälters 6 ist in Figur 2a zu sehen. Wie daraus erkennbar, ist das Innere der glockenartigen Struktur 1 gleich ausgebildet wie im Ausführungsbeispiel nach Figur 1.

Bei der handbetriebenen Pumpe aus Figur 1 sollte eine unabhängige separate Energieversorgungsquelle in der Vorrichtung vorgesehen sein. Bei der elektrisch betriebenen Pumpe aus Figu-ren 2a, 2b und 2c kann eine separate Energieerzeugung zusätzlich zu der Energieversorgung der Vakuumerzeugungsstation 11 vorgesehen sein und/oder die Dekontaminationseinheit 9 wird über die Vakuumerzeugungsstation 11 mit Spannung versorgt.

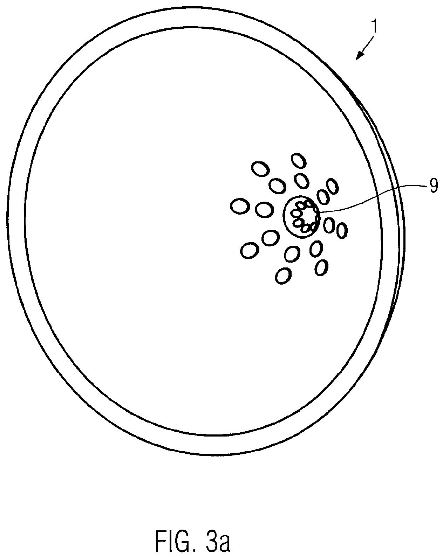

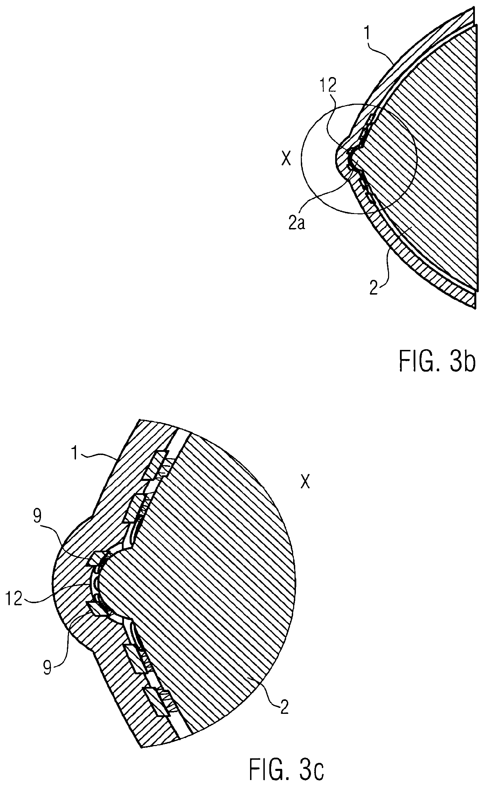

Figur 3a zeigt eine alternative Ausgestaltung einer glockenartigen Struktur 1. Die glockenartige Struktur 1 ist in diesem Fall schon so vorgeformt, dass diese an die Brust anlegbar ist. Alternativ kann diese auch so ausgebildet sein, das sie in einem nicht an der Brust angelegten Grundzustand als flaches Gebilde, das keine glockenartige Struktur aufweist, ausgebildet ist. Erst beim Anlegen an die Brust 2 bildet sich die glockenartige Struktur 1 instantan aus. Bei Entfernung von der Brust 2 kehrt das Element wieder in seinen Grundzustand zurück. Wie in der Querschnittsansicht in Figur 3b und 3c zu sehen ist, ist diese glockenartige Struktur 1 derart ausgebildet, dass diese konturabbildend an der Brust 2 anliegt. Hierzu weist die glockenartige Struktur 1 im Bereich der Brustwarze 2a eine Ausnehmung 12 auf. Innerhalb der Ausnehmung 12 sind in einer kreisförmigen Anordnung einzelne Dekontaminationseinheiten 9 angebracht. In der glockenartigen Struktur 1 außerhalb der Ausnehmung 12 sind in dem Bereich, wo die glockenartige Struktur 1 an den Warzenhof anliegt, mit einem geringen Abstand zum Warzenhof weitere Dekontaminationseinheiten 9 vorgesehen. Alle Dekontaminationseinheiten 9 können über eine gemeinsame, nicht dargestellte Steuereinrichtung angesteuert werden. Diese Steuereinrichtung sowie auch eine Energieversorgung der Dekontaminationseinheiten kann in der glockenartigen Struktur 1 integriert vorgesehen sein.

Wie in Figuren 3a und 3b dargestellt, haben die Dekontaminationseinheiten 9 im Bereich der Brustwarze eine leicht gerundete Oberfläche, um planar und im Wesentlichen vollflächig an der gerundeten Brustwarze 2a anzuliegen. Diese außerhalb der Ausnehmung 12 vorgesehenen Dekontaminationseinheiten 9 haben eine planare Fläche.

Die bei zentraler Aufsicht auf die glockenartige Struktur 1 radial am weitesten außenliegenden Dekontaminationseinheiten 9 sind an der Grenze zwischen Warzenhof und normaler Brusthaut angeordnet.

Die Dekontaminationseinheiten aus den Figuren 3a bis 3c können flächige Lichtquellen sein oder auch piezokeramische Elemente, die Ultraschallwellen ausstrahlen.

Auch wenn vorliegend für das Ausführungsbeispiel aus Figur 3 eine glockenartige Struktur 1 vorgesehen ist, kann eine ähnliche Anordnung auch innerhalb eines separaten Pads, welches in einen Büstenhalter, z.B. in eine Tasche eines Büstenhalters, eingelegt wird, vorgesehen sein.

Ein solches in einen Büstenhalter 15 eingelegtes Pad 16 ist in Figuren 4a und 4b gezeigt. Hierbei ist Figur 4b der Ausschnitt X aus Figur 4a. Das Pad 16 ist in ein Körbchen 17 des Büstenhalters 15 eingelegt. Ein solches Pad 16 kann aus einem kissenartigen, sich schwammartig verformenden Material sein. In einem nicht in den Büstenhalter 15 eingesetzten Grundzustand kann das Pad 16 als flaches kissenartiges Gebilde (z.B. mit zwei korrespondierenden Oberflächenwölbungen auf entgegengesetzten Seiten), das keine glockenartige Struktur aufweist, ausgebildet sein. Erst bei Einlegen in den Büstenhalter 15 und Anlage an der Brust bildet sich die glockenartige Struktur 1 instantan aus. Bei Entfernung aus dem Büstenhalter kehrt das Pad wieder in seinen Grundzustand zurück. Auf dessen der Brust 2 zugewandten Seite 18 ist das Pad so aufgebaut wie die glockenartige Struktur 1 in Figuren 3 a bis c. Deshalb wird auf eine erneute Beschreibung der einzelnen Elemente verzichtet. Gleiche Elemente sind mit den gleichen Bezugszeichen versehen. Zusätzlich sind aber auch Dekontaminationseinheiten 9' an der der Brust abgewandten Seite 19 des Pads 16 vorgesehen.

Auch die Dekontaminationseinheiten 9 an der der Brust abgewandten Seite 19 des Pads 16 weisen eine leicht gerundete Oberfläche auf, um planar an der Innenseite des Kröbchens 17 anzuliegen.

Die Dekontaminationseinheiten 9 an der der Brust abgewandten Seite 19 des Pads 16 sind vorteilhaft, um auch das Körbchen 17 des Büstenhalter 15 zu dekontaminieren.

Die glockenartige Struktur 1 im Ausführungsbeispiel in Figuren 3a bis 3c kann aus Silikon oder aus Kunststoff oder auch aus einem Schaumstoffmaterial sein.

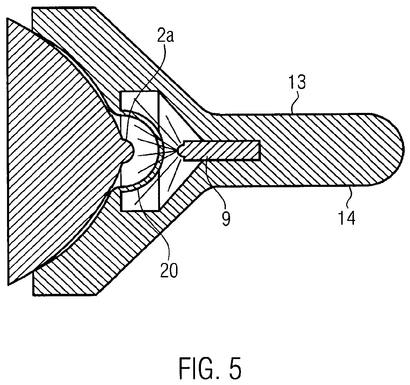

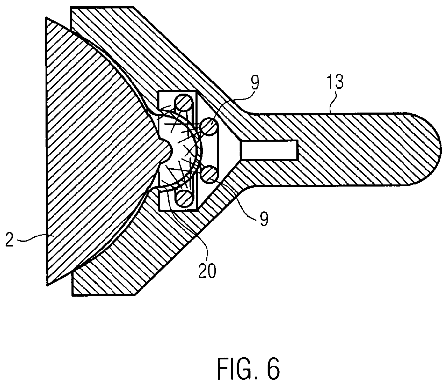

Figuren 5 und 6 zeigen zwei Ausführungsbeispiele, wobei die Vorrichtung zur Dekontamination in diesem Fall eine Vorrichtung ist, die lediglich die Funktionalität der Dekontamination hat. Das heißt, diese Vorrichtung weist nicht gleichzeitig die Funktion eines Büstenhalters beziehungsweise die Funktion einer Milchpumpe auf.

Die Vorrichtung ist als eine Art Stempel 13 ausgebildet, mit einem rückseitig vorgesehen Griffteil 14. Der Stempel 13 weist eine glockenartige Innenoberfläche auf, welche auf die Brust gestülpt wird.

Wie in Figur 5 zu sehen, ist bei dieser Ausführungsform auch ein UV-Lichtelement als Dekontaminationselement 9 gegenüber der Brustwarze 2a positioniert.

Das Ausführungsbeispiel aus Figur 6 unterscheidet sich von dem Ausführungsbeispiel aus Figur 5 dadurch, dass anstelle dieses einzigen Dekontaminationselements 9, welches gegenüber der Brustwarze ausgebildet ist, eine Mehrzahl an Dekontaminationselementen 9 vorgesehen ist, die die Brustwarze von verschiedenen Seiten behandeln.

In beiden Ausführungsbeispielen ist eine Separationswand 20 vorgesehen, welche die Dekontaminationseinheit 9 von einem Raum 21, der zwischen der Separationswand 20 und der Brustwarze 2a gebildet ist, separiert. So ist die Innenoberfläche des Stempels 13 leichter zu reinigen, und die Dekontaminationseinheit 9 kommt nicht unmittelbar mit der Brust in Kontakt. Soweit es sich bei der Dekontaminationseinheit 9 um eine UV-Lampe handelt, sollte die Separationswand 20 UV-transparent sein.

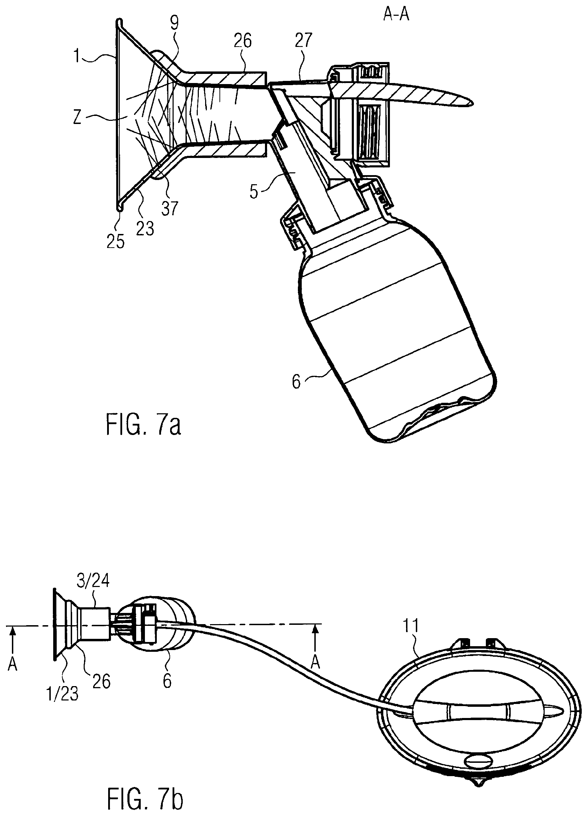

Figuren 7a bis d zeigen ein viertes Ausführungsbeispiel, bei dem die Dekontaminationseinheit 9 als Manschette außen um die glockenartige Struktur 1 und die sich daran anschließende konische Verjüngung 3 gelegt ist.

Gleiche Merkmale wie bei dem Ausführungsbeispiel der Figuren 1 bzw. 2 sind mit gleichen Bezugszeichen versehen und werden nachfolgend nicht weiter beschrieben. So sind auch bei diesem Ausführungsbeispiel ein Auffangbehälter 6 sowie eine Leitung 5 vorgesehen, über die die abgepumpte Milch zu dem Auffangbehälter 6 geführt wird. Mit Bezugszeichen 11 ist auch hier eine Vakuumerzeugungsstation vorgesehen, über die ein Vakuum zum Abpumpen von Milch erzeugt wird.

Die glockenartige Struktur 1 und die sich daran anschließende konische Verjüngung 3 sind integral und aus einem einheitlichen Material ausgebildet. Diese Elemente können auch mehrteilig ausgebildet sein. Im vorliegenden Fall ist die gesamte Kappe, welche auf den Auffangbehälter aufgesetzt, insbesondere geschraubt, als ein einheitliches Element ausgebildet. Es kann auch sein, dass lediglich die glockenartige Struktur 1 und die sich daran anschließende konische Verjüngung 3 als ein einheitliches Element ausgebildet und auf ein Basisteil 27 aufgesetzt sind. Das Basisteil 27 ist dann den Auffangbehälter aufgesetzt, insbesondere geschraubt.

Die glockenartige Struktur 1 wird im Wesentlichen durch einen vorderen, zur Brust hin gerichteten aufgeweiteten Abschnitt 23 gebildet, der nach Art eines Trichters ausgebildet ist. Von der von der Brust abgewandten Seite des aufgeweiteten Abschnitts 23 ragt nach hinten ein zylinderförmiger Tubus 24 ab. Dieser Tubus 24 wird auch als konische Verjüngung 3 bezeichnet und weist z.B. einen Durchmesser auf, der geringer ist als die Öffnung 25 des aufgeweiteten Abschnitts 23, der über die Brust gestülpt wird.

Die Dekontaminationseinheit 9 weist vorliegend ein Gehäuse 26 auf, welches als eine Art Manschette ausgebildet ist. Diese Manschette kann an einer Außenwandung des aufgeweiteten Abschnitts 23 und an einer Außenwandung des zylinderförmigen Tubus 24 anliegen und kann somit an einem Übergangsberiech zwischen den genannten Abschnitten vorgesehen sein.

In dem Gehäuse sind eine Vielzahl von einzelnen Erregungsstrahlenquellen 37 vorgesehen, deren ausgesendete Strahlung durch die in Figur 7b angedeuteten Striche verdeutlicht wird. Die Striche, die jeweils von einem gemeinsamen Basisbereich ausgehen, deuten die Richtung der Strahlung an.

In einem Bereich des Gehäuses, welcher an der Außenwandung des aufgeweiteten Abschnitt 23 anliegt, sind die Erregungsstrahlungsquellen 37 so angeordnet, dass sie schräg auf die Brust hin strahlen, d. h. schräg zu einer zentralen Achse Z der glockenartigen Struktur 1 ausgerichtet sind. Im Bereich des zylinderförmigen Tubus 24 sind die Erregungsstrahlungsquellen 37 eher derart ausgerichtet, dass diese senkrecht zur Zentralachse Z der glockenartigen Struktur ausgerichtet sind.

Das Gehäuse weist demnach einen an dem zylinderförmigen Tubus 24 montierbaren Abschnitt (vgl. Figur 7b, rechte Seite) und einen an dem aufgeweiteten Abschnitt 23 montierbaren Abschnitt (vgl. Figur 7b, linke Seite) auf. Somit ist auch das Gehäuse 26 der Dekontaminationseinheit auf einer der Brust zugewandten Seite nach Art eines Trichters ausgeformt und weist einen von diesem abgehenden zylinderförmigen Tubus auf.

Vorliegend liegt eine Innenwandung des Gehäuses 26 im Wesentlichen flächig an der Außenwandung des aufgeweiteten Abschnitts 23 und an der Außenwandung des zylinderförmigen Tubus 24 an und durchleuchten das Material aus welchem diese Abschnitte aufgebaut sind.

So ist es vorteilhaft, dass der aufgeweitete Abschnitt 23 und der zylinderförmige Tubus 24aus einem Material ausgebildet ist, welches für die Erregungsstrahlung transparent ist.

Soweit beispielsweise UV-C-Strahlung verwendet wird, sollte ein UV-C-strahlungsdurchlässiges Material verwendet werden.

Das Gehäuse 26 der Dekontaminationseinheit kann als eine Art einseitig in dessen Längsrichtung offene Manschette ausgebildet sein, die einfach auf den aufgeweiteten Abschnitt 23 und/oder den zylinderförmigen Tubus 24 mittels Schnappverbindung lösbar befestigt wird.

Jede andere Befestigungsmöglichkeit ist ebenso möglich. Es kann auch eine vollständig geschlossene Manschette gebildet sein, welche z.B. von hinten auf den zylinderförmigen Tubus 24 aufgefädelt wird. Hierzu ist es vorteilhaft, den zylinderförmigen Tubus 24, mit einem oder ohne einen daran integral gebildeten aufgeweiteten Abschnitt 23, als separates Element auf das Basisteil 27 aufzusetzen. Nachdem dieses vollständig in dessen Umfangsrichtung geschlossene Gehäuse dann aufgesetzt ist, kann dieses Ensemble dann wieder an dem Basisteil 27 befestigt werden.

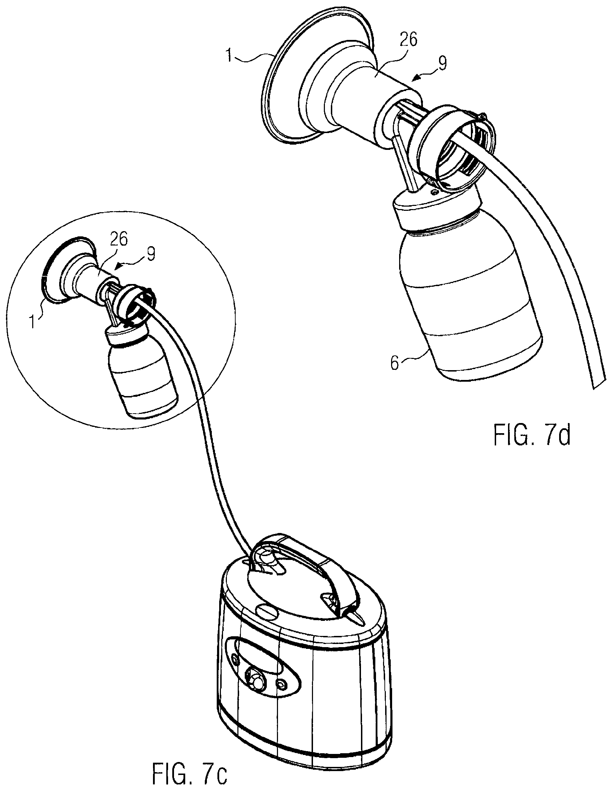

In Figur 7c und 7d sind dreidimensionale Ansichten des Ausführungsbeispiels aus Figuren 7a und 7b gezeigt, wobei die manschettenartige Ausgestaltung des Gehäuses 26 der Dekontaminationseinheit zu erkennen ist.

Die Elektronik zum Betrieb der Dekontaminationseinheit kann in dem Gehäuse 26 vorgesehen sein, oder über Leitungen, die aus dem Gehäuse 26 herausragen mit einer Steuervorrichtung bzw. Energieversorgungseinrichtung verbindbar sein.

Das Ensemble aus zylinderförmigem Tubus 24 mit einem daran integral gebildeten aufgeweiteten Abschnitt 23 weist bei der vorliegenden Ausgestaltung keine elektronischen Komponenten auf. Vorteilhafterweise weist auch das Basisteil 27 und der Auffangbehälter 6 keine elektronischen Komponenten auf, d. h. die einzigen elektronischen Komponenten, welche bei demontierter Dekontaminationseinheit vorgesehen sein können, sind diejenigen der Vakuumerzeugungsstation 11.

Somit kann das Ensemble aus zylinderförmigem Tubus 24 und/oder aufgeweitetem Abschnitt 23 und/oder Basisteil 27 und/oder Auffangbehälter 6 zusammen oder separat und bevorzugt flüssig oder mit Dampf gereinigt werden, ohne dass es zu Problemen mit der Elektronik kommt.

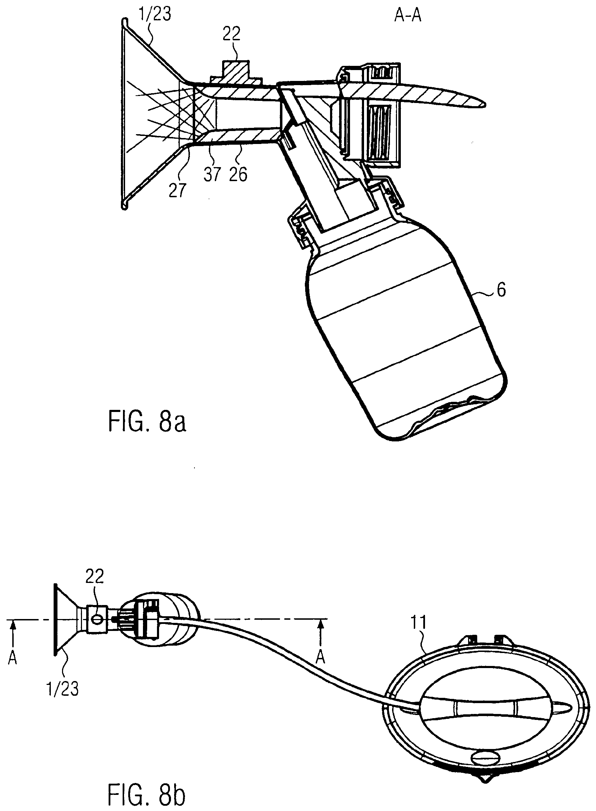

In Figuren 8a bis d ist ein fünftes Ausführungsbeispiel dargestellt, bei dem die Dekontaminationseinheit 9 im Inneren der konischen Verjüngung 3 eingesetzt ist.

Im Unterschied zu dem Ausführungsbeispiel aus Figuren 7a bis d ist bei dem fünften Ausführungsbeispiel das Gehäuse der Dekontaminationseinheit 9 nicht radial außen an dem Ensemble aus zylinderförmigem Tubus 24 und aufgeweitetem Abschnitt 23 vorgesehen, sondern das Gehäuse 26 ist in den zylinderförmigen Tubus 24 eingeschoben.

Das Gehäuse 26 weist an einem vorderen, der Brust zugewandten Abschnitt Erregungsstrahlungsquellen 37 auf. Die Erregungsstrahlungsquellen 37 sind entlang eines gerundeten Wandungsabschnitts, der das Gehäuse zur Brust hin in einer trompetenartigen Ausgestaltung abschließt, angeordnet, so dass sie in einem Winkel schräg zur Zentralachse der glockenartigen Struktur auf die Brust einstrahlen.

Die in der Figur 8a am weitesten rechts vorgesehenen Erregerstrahlungsquellen 27 sind im Verhältnis zu den in Figur 8a weiter links vorgesehenen Strahlungsquellen senkrechter zu der Zentralachse Z ausgerichtet, wobei die in dem Gehäuse weiter links vorgesehenen Strahlungsquellen eher schräger stehen.

Wenn für die Dekontaminationseinheit 9 ein solcher Einsatz verwendet wird, ist es günstig, die Energieversorgung durch ein Induktionselement 22 vorzusehen und nicht über eine Verkabelung, die beispielsweise dann durch die glockenartige Struktur 1 hindurch gehen muss, zu gewährleisten, sondern es ist vorteilhafterweise ein Induktionselement 22 vorzusehen. Vorliegend ist das Induktionselement 22 an der Außenwandung des zylinderförmigen Tubus 24 vorgesehen.

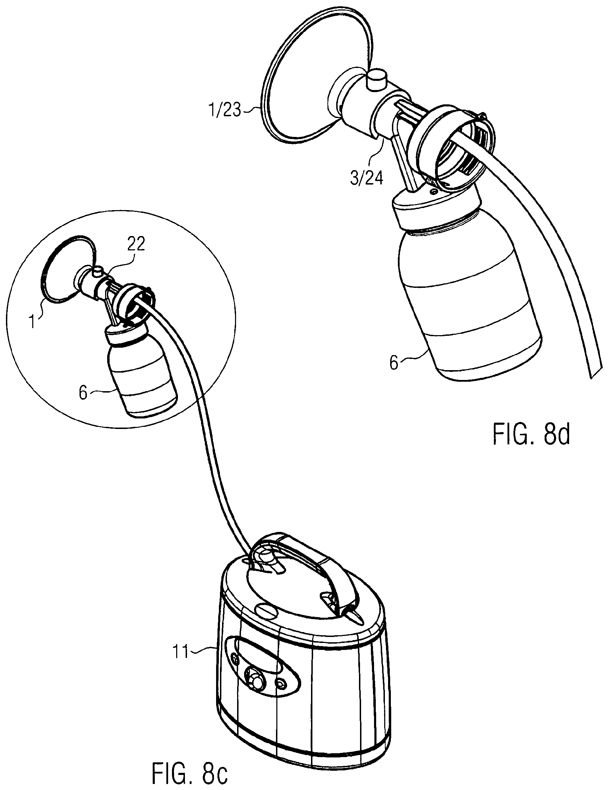

Dieses Induktionselement 22 kann fest und unverlierbar an dem zylinderförmigen Tubus 24 und/oder dem aufgeweiteten Abschnitt 23 vorgesehen sein oder aber auch als eine Art Manschette (vgl. Fig. 8c und d), deren Querschnitt z.B. C-förmig ausgestaltet ist, vorgesehen sein, und auf den aufgeweiteten Abschnitt 23 bzw. den zylinderförmigen Tubus 24 aufgesteckt werden. So kann ein einfacher modularer Aufbau bereitgestellt werden, wobei die Spannungs- bzw. Leistungsversorgung der Erregungsstrahlungsquellen, welche in dem Gehäuse 26 vorgesehen sind, physikalisch getrennt durch die Wand des aufgeweiteten Abschnitts 23 bzw. des zylinderförmigen Tubus 24 angeordnet ist.

Auch bei diesem Ausführungsbeispiel kann das Ensemble aus zylinderförmigem Tubus 24 mit einem daran integral gebildeten aufgeweiteten Abschnitt 23 keine elektronischen Komponenten aufweisen. Vorteilhafterweise weisen auch das Basisteil 27 und der Auffangbehälter 6 keine elektronischen Komponenten auf, d. h. die einzigen elektronischen Komponenten, welche bei demontierter Dekontaminationseinheit vorgesehen sein können, sind diejenigen der Vakuumerzeugungsstation 11. Somit kann das Ensemble aus zylinderförmigen Tubus 24 und/oder aufgeweitetem Abschnitt 23 und/oder Basisteil 27 und/oder Auffangbehälter 6 zusammen oder separat gereinigt werden, ohne dass es zu Problemen mit der Elektronik kommt.

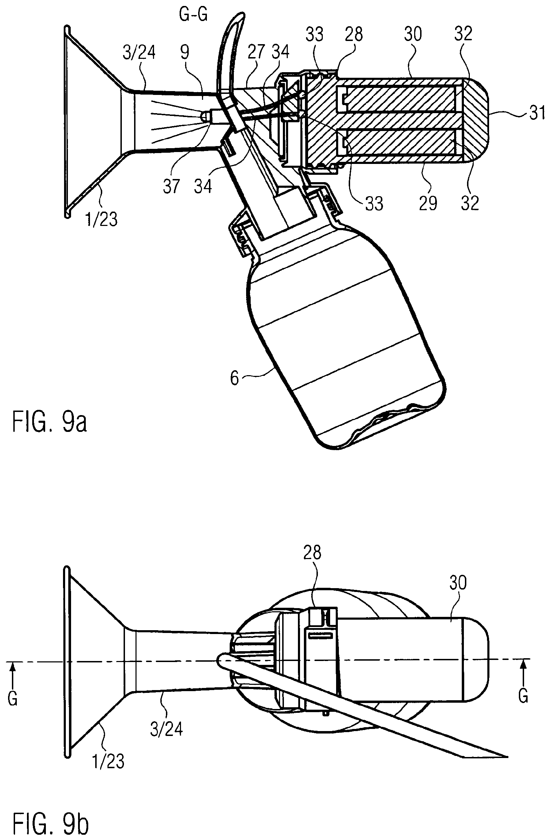

In Figuren 9a und 9b ist ein sechstes Ausführungsbeispiel dargestellt, bei dem ein Batteriepack wiederholt lösbar rückseitig an der Dekontaminationsvorrichtung vorgesehen ist

In diesem Ausführungsbeispiel ist an dem Basisteil 27 rückseitig ein Mittel zum lösbaren Verbinden mit einer Spannungsversorgung 28 vorgesehen. Die Spannungsversorgung 28 wird vorliegend durch ein Batteriepack 29 gewährleistet. Dieses Batteriepack 29 weist ein Gehäuse 30 und eine Kappe 31 auf. In dem Gehäuse 30 des Batteriepacks 29 sind Ausnehmungen vorgesehen, in welche Batterien 32 einsetzbar sind. Nachdem die Batterien 32 in die Ausnehmung eingesetzt sind, wird das Gehäuse 30 mit der Kappe 31 verschlossen, so dass das Batteriepack 29 als einheitliches Element gebildet wird.

Vorderseitig an dem Batteriepack sind in der Figur 9a nicht dargestellte Kontakte ausgebildet, die mit Kontaktelementen 33, die rückseitig an dem Basisteil 27 vorgesehen sind, kontaktiert werden können.

Die Erregungsstrahlungsquelle 37 (im vorliegenden Ausführungsbeispiel eine einzige) ist über die in Figur 9a schematisch dargestellten Leitungen 34, die durch das Basisteil 27 verlaufen, mit dem Batteriepack 29 verbunden. Es können in der Dekontaminationseinheit 9, wie in den Beispielen aus Figuren 7 und 8, auch mehrere Erregungsstrahlungsquellen 37 und nicht nur eine einzige Erregungsstrahlungsquelle 37 vorgesehen sein.

Vorliegend ist die Erregungsstrahlungsquelle 37, die die Dekontaminationseinheit 9 bildet, integral an dem Basisteil 27 vorgesehen. Das Basisteil 27 kann als separates Element zwischen dem Ensemble aus zylinderförmigem Tubus 24 und aufgeweitetem Abschnitt 23, und dem Auffangbehälter 6 eingebaut werden.

An dieses Basisteil 27 kann dann in lösbarer und mehrmals ankoppelbarer Weise das Batteriepack 29 angeschlossen werden. Vorliegend ist eine Schraubverbindung zwischen dem Batteriepack 29 und dem Basisteil 27 vorgesehen, das als Mittel zur lösbaren Verbindung mit der Spannungsversorgung 28 dient.

In dem Ausführungsbeispiel aus den Figuren 9a, b ist die Dekontaminationseinheit, insbesondere die Erregungsstrahlungsquelle 37, in einer Zentralachse der glockenartigen Struktur 1 vorgesehen und an einer Rückwand, welche durch eine Wandung des Basisteils 27 gebildet wird.

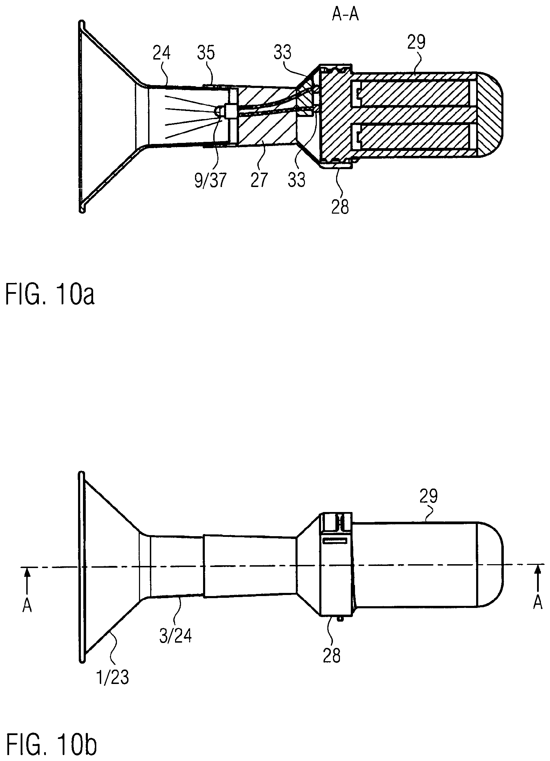

In Figuren 10a und 10b ist eine weitere Ausgestaltung der erfindungsgemäßen Vorrichtung dargestellt.

Auch in diesem Fall ist die Dekontaminationseinheit, d. h. vorliegend eine einzige in Erregungsstrahlungsquelle 37, integral in dem Basisteil 27 vorgesehen. Das Basisteil 27 hat rückseitig, d.h. an einer von der Brust abgewandten Seite (vgl. Figur 10a rechte Seite) dieselbe Ausgestaltung wie das Basisteil in Figur 9a. Folglich sind auch hier Kontaktelemente 33 vorgesehen über welche ein Batteriepack 29 kontaktierbar ist.

An seiner der Brust zugewandten Seite weist das Basisteil 27 einen zylinderförmigen Randabschnitt 35 auf, der im Durchmesser im Vergleich zu dem zylinderförmigen Tubus 24 aufgeweitet ist, so dass eine Steckverbindung zwischen dem zylinderförmigen Tubus 24 und dem Basisteil 27 gewährleistet ist. Auch vorliegend ist der zylinderförmige Tubus 24 integral mit dem aufgeweiteten Abschnitt 23 ausgebildet.

Das gesamte Ensemble aus separatem Basisteil 27 und dem zylinderförmigem Tubus 24 mit dem daran integral vorgesehenen aufgeweiteten Abschnitt 23 bildet eine Art Brusthaubeneinheit. Der von dem Tubus 24 mit dem daran integral vorgesehenen aufgeweiteten Abschnitt 23 gebildete vordere Teil ist von dem durch den Basisteil 27 gebildeten hinterem Teil der Brusthaube separierbar. Diese Brusthaube ist an das Batteriepack 29 bzw. ein Gehäuseteil der Vorrichtung ankoppelbar.

Die Dekontaminationseinheit bzw. die Erregungsstrahlungsquelle ist vorliegend im Wesentlichen in der Zentralachse der glockenartigen Struktur vorgesehen.

Auch wenn bei den Ausführungsbeispielen aus Figur 9a und b bzw. 10a und 10b lediglich eine einzige Erregungsstrahlungsquelle vorgesehen ist, können auch hier, wie in den Ausführungsbeispielen aus Figuren 8 und 7, mehrere Erregungsstrahlungsquellen vorgesehen sein.

Bei den Ausführungsbeispielen in Figuren 7 und 8 sind hingegen die Erregungsstrahlungsquellen nicht an einem rückwärtigen Teil und in einer Zentralachse der glockenartigen Struktur vorgesehen, sondern an einer Außenwandung um die glockenartige Struktur herum angeordnet.

Das Ausführungsbeispiel in Figur 11a und 11b stellt eine Weiterbildung des Ausführungsbeispiels in Figuren 4a und 4b dar. Deshalb sei hier für die weiteren Details auf die Beschreibung zu Figur 4a und 4b verwiesen.

Anstelle der pad- bzw. kissenartigen Struktur kann die glockenartige Struktur 1 auch aus einem formstabilen z.B. harten Material ausgebildet sein, welches insbesondere derart konfiguriert ist, dass es bei Anlegen eines Vakuums nicht bzw. nur unwesentlich kollabiert. Damit ein Vakuum angelegt werden kann, ist es günstig, dass die glockenartige Struktur vakuumdicht ist, und auch dichtend an die Brust angelegt werden kann. Die glockenartige Struktur kann so durch eine Art formstabile Brusthaube gebildet werden.

Darüber hinaus ist bei dem vorliegenden Ausführungsbeispiel in Figur 11b und 11a in dem Pad bzw. Kissen ein Schlauchstutzen 36 vorgesehen, der in der Ausnehmung in dem Pad, welches den Warzenhof umgibt, endet. Über diesen Schlauchstutzen 36 kann eine Pumpe angeschlossen werden, so dass in der Ausnehmung ein Vakuum erzeugt und somit Milch von der Brust abgepumpt wird. Dank der Ausgestaltung, bei welcher der Schlauchstutzen in dem Pad bzw. dem Kissen vorgesehen ist, ist es möglich, eine sehr unauffällige Pumpvorrichtung anzubieten, wobei während des Pumpens der BH angelassen werden kann und es überhaupt nicht gesehen wird, dass eine Dekontamination und/oder ein Pumpen stattfindet.

Zusätzlich zu der beschriebenen Dekontaminationseinheit bzw. der Erregerstrahlungsquelle, kann in der Vorrichtung auch ein Feuchtigkeitssensor vorgesehen sein, mittels welchem z.B. ein Milch- bzw. Flüssigkeitsaustritt detektiert wird.

Die Steuervorrichtung die die Dekontaminationseinheit bzw. die Erregerstrahlungsquelle(n) steuert kann z.B. auch eine Funktionalität aufweisen, dass bei Detektion von Flüssigkeit bzw. einem bestimmten Feuchtigkeitsgrenzwert die Dekontaminationseinrichtung bzw. die Erregerstrahlungsquelle(n) aktiviert wird/werden.

### Bezugszeichenliste

- 1: glockenartige Struktur
- 2: Brust
- 2a: Brustwarze
- 3: konische rohrartige Verjüngung
- 4: der Brustwarzen gegenüberliegende Seite
- 5: Leitung
- 6: Auffangbehälter
- 7: Schraubdeckel
- 8: Hebel
- 9, 9': Dekontaminationseinheit
- 10: Warzenhof
- 11: Vakuumerzeugungsstation
- 11a: Vakuumschlauch
- 12: Ausnehmung
- 13: Stempel
- 14: Griffteil
- 15: Büstenhalter
- 16: Pad
- 17: Körbchen
- 18: der Brust zugewandte Seite
- 19: der Brust abgewandte Seite
- 20: Separationswand
- 21: Raum
- 22: Induktionselement
- 24: zylinderförmiger Tubus
- 23: aufgeweiteter Abschnitt
- 27: Basisteil
- 25: Öffnung
- 26: Gehäuse
- 37: Erregungsstrahlungsquelle
- 28: Mittel zur lösbaren Verbindung mit einer Spannungsversorgung
- 29: Batteriepack
- 30: Gehäuse
- 31: Kappe
- 32: Batterie
- 33: Kontaktelement
- 34: Leitung
- 35: zylinderförmiger Randabschnitt
- 36: Schlauchstutzen

## Patentansprüche

1. Tragbare Vorrichtung zur Dekontamination einer Brust (2),
wobei jeweils zumindest eine Dekontaminationseinheit (9, 9') auf einer der Brust (2) zugewandten Seite (20) und auf einer der Brust (2) abgewandten Seite (19) der Vorrichtung angeordnet ist wobei die Dekontaminationseinheit (9) auf der der Brust (2) zugewandten Seite (20) die Brust (2) zumindest mittels einer physikalischen Methode dekontaminieren,
und wobei
die Dekontaminationseinheiten (9) in einem Pad bzw. Kissen aus einem sich verformenden Material vorgesehen ist,
welches als separates Element in einen Büstenhalter einlegbar ist,
wobei das Pad bzw. Kissen derart konfiguriert ist, dass es in einem Grundzustand, in dem es nicht in einen Büstenhalter eingesetzt ist, als flaches kissenartiges Gebilde ausgeformt ist, wobei sich beim Einlegen in den Büstenhalter eine glockenartige Struktur ausbildet.

2. Vorrichtung nach Anspruch 1, **dadurch gekennzeichnet, dass** das Pad bzw. Kissen mit einem Schlauchstutzen (36) versehen ist, über welchen eine Pumpe anschließbar ist, um Milch von der Brust abzupumpen.

3. Vorrichtung nach einem der vorherigen Ansprüche, **dadurch gekennzeichnet, dass** diese eine Steuereinrichtung aufweist, welche die Dekontaminationseinheit (9) ein und/oder ausschaltet, und/oder die Intensität reguliert.

## Claims

1. Portable device for decontaminating a breast (2),
wherein respectively at least one decontamination unit (9, 9') is arranged on a side (20) of the device facing the breast (2) and on a side (19) of the device facing away from the breast (2),
wherein the decontamination unit (9) on the side (20) facing the breast (2) decontaminates the breast (2) at least by means of a physical method, and
wherein the decontamination units (9) are provided in a pad or cushion made of a deformable material, which can be inserted as a separate element into a bra, wherein
the pad or cushion is configured such that, in a basic state in which it is not inserted into a bra, it is shaped as a flat cushion-like structure, wherein, when inserted into the bra, a bell-like structure is formed.

2. Device according to claim 1, **characterized in that** the pad or cushion is provided with a hose connection (36) via which a pump can be connected to pump milk from the breast.

3. Device according to one of the previous
claims, **characterized in that** it has a control device which switches the decontamination unit (9) on and/or off and/or regulates the intensity.

## Revendications

1. Dispositif portable pour la décontamination d'un sein (2),
où au moins une unité de décontamination (9, 9') est disposée respectivement sur un côté (20) du dispositif tourné vers le sein (2) et sur un côté (19) du dispositif détourné du sein (2), où l'unité de décontamination (9) décontamine le sein (2) sur le côté (20) tourné vers le sein (2) au moins au moyen d'une méthode physique,
et où
les unités de décontamination (9) sont prévues dans un tampon ou un coussin en un matériau déformable,
qui peut être inséré comme élément séparé dans un soutien-gorge,
où le tampon ou le coussin est configuré de telle manière que dans un état de base, dans lequel il n'est pas inséré dans un soutien-gorge, il est formé comme une structure plate de type coussin, où une structure en forme de cloche se forme lors de l'insertion dans le soutien-gorge.

2. Dispositif selon la revendication 1, **caractérisé en ce que** le tampon ou le coussin est pourvu d'un raccord de tubulure (36) par laquelle une pompe peut être raccordée pour extraire du lait du sein.

3. Dispositif selon l'une des revendications précédentes, **caractérisé en ce qu'**il comporte un équipement de commande qui active et/ou désactive l'unité de décontamination (9), et/ou régule l'intensité.
